# EUROPEAN PATENT SPECIFICATION

(11) **EP 2 521 485 B1**
(45) Date of publication and mention of the grant of the patent: **20.03.2024**
(21) Application number: 11731723.0
(22) Date of filing: 10.01.2011
(51) Int. Cl.: A61B 5/282, A61B 5/11, A61B 5/00

(54) **PHYSIOLOGICAL SIGNAL COLLECTION APPARATUS AND PERFORMANCE MONITORING APPARATUS INCORPORATING SAME**
VORRICHTUNG ZUR ERFASSUNG PHYSIOLOGISCHER SIGNALE UND LEISTUNGSÜBERWACHUNGSVORRICHTUNG DAMIT
APPAREIL DE COLLECTE DE SIGNAUX PHYSIOLOGIQUES ET APPAREIL DE CONTRÔLE DE PERFORMANCES COMPRENANT CELUI-CI

(30) Priority: 08.01.2010 HK 10100226
(43) Date of publication of application: 14.11.2012
(73) Proprietor: Dayton Technologies Limited, New Territories, Hong Kong (CN)
(72) Inventor: YUEN, Paul Anthony, New Territories Hong Kong (CN)
(74) Representative: Abel & Imray LLP
(86) International application number: PCT/IB2011/050078
(87) International publication number: WO 2011/083441

(56) References cited:
- JP-A- 2002 143 110
- US-A1- 2005 049 515
- US-A1- 2005 096 556
- US-A1- 2005 096 556
- US-A1- 2007 093 707
- US-A1- 2007 285 868
- US-A1- 2008 284 650
- US-A1- 2009 048 540
- US-A1- 2009 069 724

## Description

### Field of the Invention

The present invention relates to physiological signal collection apparatus, and more particularly to chest-worn physiological signal collection apparatus. This invention also relates to performance monitoring apparatus comprising a physiological signal collector and a signal processing device for processing collected physiological signals. More specifically, although not solely limited thereto, the present invention also relates to chest-worn swim monitors.

### Background of the Invention

Physiological signal collectors are useful for collecting physiological data of a person during physical exercises, during medical examination or during everyday life. Typical physiological signals which are commonly collected for processing and analyzing include ECG, heart rate, blood pressure, blood oxygen content, body temperature. The collected signals are typically processed and converted into data which provide information on the state of health, physical fitness, or physical performance of a person.

US2007/0285868 discloses a sensor for measuring a sensor for measuring a physiological signal and a method for manufacturing such a sensor. The sensor comprises a flexible substrate and at least one electrode having a signalling surface and a moisture insulated signal transmission conductor, which is connected electrically to the electrode.

A chest strap is a known type of physiological signal collectors adapted to be chest-worn by a person for collecting physiological signals during physical exercises or activities. Physiological signals commonly collected by a chest strap include, for example, heart-rate, ECG pulses, skin conductivity, infra-red absorption or other electrical or opto-electrical signals measureable from the skin of a person.

A typical chest strap usually includes a plurality of electrodes or sensors which is mounted on a flexible plastic chest strap in a spaced apart manner to collect weak physiological signals in electrical or optical form from a human body for differential signal processing. Each electrode typically comprises a signal reception pad having a signal reception surface with a surface area large enough to collect signals which are strong enough for processing by a signal processing device, such as a heart rate signal processor.

The flexible plastic strap is usually a pre-assembled strap loop which comprises a length adjustment or tension adjustment arrangement to tighten the strap against the body of a person, usually the chest, during use to provide electrical contact between the electrodes and the skin of a user. The strap is usually made of soft plastics to provide flexibility for body wearing and electrical insulation between the electrodes. The tensions adjustment arrangement typically comprises a length of soft plastic strap portion which runs around buckle or clasp arrangements.

However, such chest-type physiological data collectors are uncomfortable and difficult to wear and adjust. Also, it is noted that known chest straps do not operate well enough for wet applications, for example swimming or water sports.

Therefore, it would be advantageous to provide improvement chest straps to mitigate shortcoming aspects of conventional chest straps.

### Summary of the Invention

According to the present invention, there is provided a swim chest strap comprising a first ECG signal collection electrode and a second signal collection electrode for collection of ECG signals in conductive water such as sea water or chlorinated water during swimming or water sports, wherein each signal collection electrode comprises a signal collection pad having a skin contact portion, a signal output portion comprising a signal output pad which includes a signal output terminal, and an elongate bridging portion interconnecting the signal collection pad and the signal output pad, wherein the signal collection pad, the signal output pad and the bridging portion are integrally moulded of carbonized rubber which is a flexible, conductive and resilient material; wherein the signal output portion is exposed for making external electrical contact and comprises a signal output terminal for outputting collected ECG signals; and wherein resistance between the skin contact portion and the signal output terminal is less than 500 ohms.

There is disclosed a physiological signal collection electrode for collecting ECG signals in or under water, comprising a signal collection pad having a skin contact portion, a signal output pad, and an elongate bridging portion interconnecting the signal collection pad and the signal output pad, wherein the signal collection pad, the signal output pad and the bridging portion are integrally moulded of a flexible, conductive and resilient material; characterized in that the width of the bridging portion is substantially smaller than that of the skin contact portion, and resistance between the signal collection pad and the signal output pad is less than 1 kΩ to facilitate collection of ECG signals in or under water during swimming or water sports.

A narrowed bridging portion operates to concentrate collected physiological signals collected by the skin contact portion before the signals are output to the signal output pad. An elongate bridging portion reduces skin covering area for better wearer comfort as well as providing better resiliency to the electrode when the bridging portion is extended. The signal collection pad coupled with the narrowed bridging portion makes the electrode more flexible and is therefore particularly suitable for collecting human ECG signals, for example, from rib cage regions of a person.

To mitigate spurious signals reception of the bridging portion, the bridging portion may be covered by a flexible, resilient and waterproof insulating material such as rubber, silicone rubber or soft plastics. The insulating material is preferably over-moulded on the electrode for better shielding against spurious signals.

The skin contact portion may elevate from the signal collection pad and may be surrounded by an insulating material. The surface of the insulating material is preferably flush with the surface of the skin contact portion, thereby making a continuous flush surface for better wear comfort while maintaining a well defined signal collection area.

The signal output pad may include a signal output terminal to output the collected physiological signal for external processing.

The effective resistance of the electrode measured between the skin contact portion and the signal output terminal is less than 500Ω, and preferably less than 400Ω. It has been found that such a low resistance is beneficial for in or under water applications. Accordingly, the electrode may be configured as an ECG signal collector for collecting ECG signals during swimming.

The skin contact surface may be flexible and elongate with an effective skin contact surface of between 7.5 cm² to 20cm².

The skin contact portion may be between 1.5-2.5 cm wide and 5-8 cm long.

The narrowed bridging portion may be between 6 and 10 cm long and about half or less than half of the width of the skin contact portion.

The flexible, conductive and resilient material may be selected form carbonized rubber, carbonized fabrics, Nickel copper plated polyester, silver nylon mesh, cotton silver bamboo fiber, or other conductive fabrics such as metalized fabrics or the like.

There is provided an ECG signal collection strap comprising first and second ECG signal collection electrodes which are respectively mounted at first and second free longitudinal ends of an insulating strap, wherein each signal collection electrode comprises a signal output terminal and a signal collection pad having a skin contact surface which is adapted for collecting ECG signals from the skin of a user, characterized in that, the ECG signal collection electrode is an electrode of the present invention.

There is provided an ECG signal collection strap comprising first and second ECG signal collection electrodes which are respectively mounted at first and second free longitudinal ends of an insulating strap, wherein each signal collection electrode comprises a signal output terminal and a signal collection pad having a skin contact surface which is adapted for collecting ECG signals from the skin of a user, characterized in that, first and second mechanical fastening parts are provided respectively at the first and second longitudinal ends of the strap for converting the longitudinal strap into a strap loop for wearing on the body of a user, and the first and second mechanical fastening parts are arranged such that the strap will urge the skin contact surfaces of the first and second electrodes to abut skin of the user to make electrical contacts and to collect physiological signals when the strap loop is worn on the body of the user with the fastening parts engaged.

Such an arrangement facilitates front wearing of the strap as a user could engage the fastening parts at the front of the chest.

The signal output terminal comprises a mechanical mating portion of a conductive mechanical fastening part which is adapted for engagement with a complementary mechanical mating portion of a counterpart mechanical fastening part. A signal output terminal which is also a mechanical portion of a conductive mechanical fastening part means added user convenience because a user only needs to make a single connection step to achieve both electrical and mechanical connection.

The first and second mechanical fastening parts of the signal collection strap may be adapted for mechanical engagement with a portable ECG signal processing device, and to form a complete strap loop upon engagement with the portable ECG signal processing device, and wherein the strap is arranged such that ECG signals collected by the electrodes are delivered to the portable ECG signal processing device via the mechanical fastening parts. The portable ECG signal processing device is useful for ECG signal processing as well as serves as a latching bridging for converting the strap into a strap loop.

The signal output terminal may also a conductive mating portion of a snap fastener part. This facilitates snap fastening for making both electrical and mechanical connections for added convenience.

The insulating strap may be elastic and its length may be adjustable. The insulating strap may be made of air and/or moisture permeable elastomeric fabric such as elastomeric polyamide or swimwear fabric such as Nylon, Lycra^{®}, polyester, or the like. Anti-slid texture may be formed on portions of the insulating strap which are in contact with user skin during use.

The bridging portion of each of the electrodes may be embedded within the insulated strap. The signal collection pad of each of the electrodes may be bonded on the insulated strap.

The mechanical fastening components may be arranged to convert the longitudinal tensioning strap into a tensioning strap loop The conductive parts of the mechanical fastening components may form part of the signal output terminals and/or vice versa.

The swim motion detector and the ECG signal processing device may be formed as a module and may be housed within a common housing, and the ECG signal processing device has first and second signal input terminals. The ECG signal processing device may be arranged to obtain ECG signals from the strap via the signal output terminals of the strap.

The swim motion detector may comprise a 3-axis accelerometer which is arranged to collect swim motion data.

The effective signal path resistance between the skin contact portion of the electrode and a corresponding output terminal may be less than that of the swim water signal path between the skin in contact with the electrode and the corresponding output terminal.

The monitor is adapted for use in sea water, chlorinated water or other ionized water, and the effective signal path resistance of each of the electrodes is below 800 ohms, preferably below 500 ohms.

Each of the first and second terminals of the ECG processing device may be formed as a mating portion of a mechanical fastener part, and each of the output terminals of the strap is formed into a complementary mating portion of a counterpart mechanical fastener part.

The physiological signal processing module may connect with the signal collection strap to form a complete strap loop when they are mechanically engaged.

The strap loop is a body strap such as a chest strap, head strap, arm strap, thigh strap, or the like.

### Brief Description of the Drawings

Embodiments of the present invention will be explained below by way of example and with reference to the accompanying drawings, in which
Figure 1 is a perspective view of a chest strap illustrating a first embodiment of the present invention,
Figures 2 and 3 respectively show the front and rear views of the chest strap of Figure 1,
Figure 4 shows the strap of Figures 1-3 when fastened with an ECG processing apparatus,
Figure 4A shows the strap of Figure 4 when the ECG processing apparatus partly detached from the chest strap,
Figure 5 is a schematic view illustrating the chest strap of Figure 1 in use,
Figure 5A is an enlarged view showing the chest strap of Figure 1 in combination with an ECG signal processor,
Figure 6 is a plan view of a first embodiment of an ECG electrode covered with an insulator,
Figures 6A & 6B are respectively plan and side views of the electrode alone,
Figure 6C is a schematic side view illustrating the electrode of Figure 6 connected with an output terminal,
Figure 7 shows a second embodiment of an ECG electrode, and
Figure 7A shows a cross-sectional view of the electrode of Figure 7.

### Detailed Description of Exemplary Embodiments

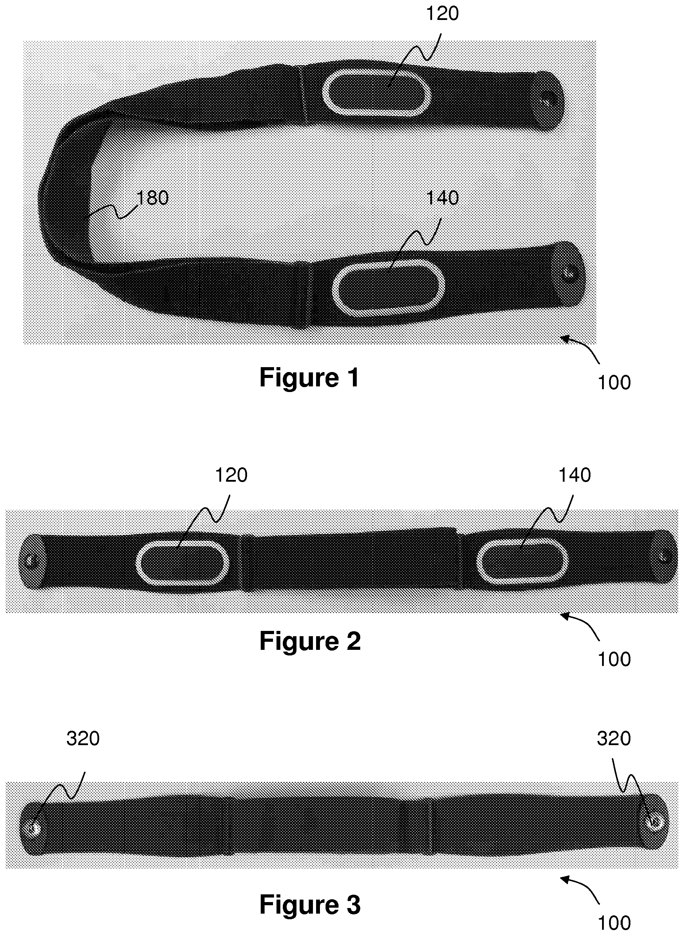

The strap of Figures 1 to 5A is a chest-worn ECG signal collection strap apparatus (more commonly known to as a 'chest strap') illustrating an exemplary embodiment of a physiological signal collection strap of the present invention. The chest strap 100 comprises first 120 and second 140 ECG signal collection electrodes which are mounted on an elastic strap 180. The elastic strap is made of a breathable and insulating material, such as a fabric comprising a mixture of polyamide and an elastomer. Each of the signal collection electrodes 120, 140 comprises a signal collection pad and a signal output portion. Each signal collection pad comprises a flexible signal collection surface which is arranged to contact the skin and collect ECG signals from a user during use.

A carbonized rubber pad as an example of a flexible signal collection pad is used to collect ECG signals from the chest of a user. The flexible signal collection pad is arranged such that it is urged and pressed against the skin of a user by the tension, more particularly the body-bound tension, of the elastic strap during use when the elastic strap is tightened on the body of the user to minimize contact resistance between the skin and the signal collection pad. The flexible collection surface are arranged to transmit collected ECG signals to a signal output portion which is terminated at a conductive fastening component located at a longitudinal end of the strap. The conductive fastening components of the two ECG signal collection electrodes are located at opposite longitudinal ends of the strap and are arranged to convert the longitudinal strap into a strap loop when the fastening components are respectively anchored onto corresponding or counterpart fastening components. Each conductive fastening component comprises a mechanical mating portion which is adapted for making counterpart engagement with another compatible mechanical mating portion of a compatible fastener. Example of suitable fasteners includes a snap fit fastener or a magnetic clasp.

Each of the signal collection electrodes is mounted on the insulating fabric strap with the signal output portion located at or proximal to a free longitudinal end of the strap such that the two signal output portions are located at the two extreme or free longitudinal ends before the chest strap is converted into a chest strap loop. In this condition, the two signal collection pads are located intermediate the two conductive fasteners which are located at the free longitudinal ends of the strap. To provide good mechanical fastening and good electrical contact, the signal output portion of the signal collection electrode is riveted onto the chest strap by a rigid conductive fastener, which conductive fastener also operates as a signal output terminal to deliver ECG signals from the chest strap for further processing.

In order that ECG signals can be effectively collected from the chest of a user, the signal collection pads of the two ECG signal collection electrodes are disposed on the strap such that when the fastening components are mechanically connected with the respective counterpart or corresponding fastening means to form a tensioned strap loop and duly worn and aligned on the chest of a user, the signal collection surfaces will be attached respectively to the left and right portions of the rib cage of the user. To adapt for this application, the distance between the two signal collection surfaces is equal to or approximately equal to the distance between the portions of the rib cage left and right of the sternum when the chest strap loop is duly worn. In order to provide adequate separation between the two signal collection surfaces during use, the signal collection pad of a signal collection electrode is connected to its corresponding signal output portion via a bridging portion which is adapted to provide spatial separation between the signal collection surface and the signal output portion. For good signal continuity, the signal collection pad, the bridging portion and the output portion are integrally moulded of carbonized rubber as an example of a flexible and conductive substance. It will be noted that the moulded flexible electrode is substantially planar for wearing comfort. The chest strap also comprises a strap length adjustment mechanism for adjusting the effective or usable length of the chest strap to cater for users of different chest widths.

Construction of an exemplary chest strap will be described in more detail below. The elastic strap comprises first and second elongate fabric layer strips which are glued together to form a double-layered strap. Each of the fabric layers are made of an insulating and elastic fabric, such as a mixture of polyamide and elastomer. A breathable fabric which is moisture permeable is preferred for comfort wearing. The planar electrode is sandwiched or embedded between the two fabric layers, while leaving the signal collection surface and the signal output portion exposed for external electrical contact. The regions of the unexposed planar electrode are also glued to the elastic strap for durability. A bonding agent is applied to the periphery of the exposed signal collection surface to further bond the signal collection pad to the strap and to provide a smooth edge. As an optional feature, the back surface of the elastic strap, which is the surface adapted to be in contact with the skin of a user, is formed with a woven anti-slid texture. As a further option, the material composition of the two strap layers could be different. For example, the textured layer may be made of 37% elastomer while the non-textured layer comprises less than 20% elastomer.

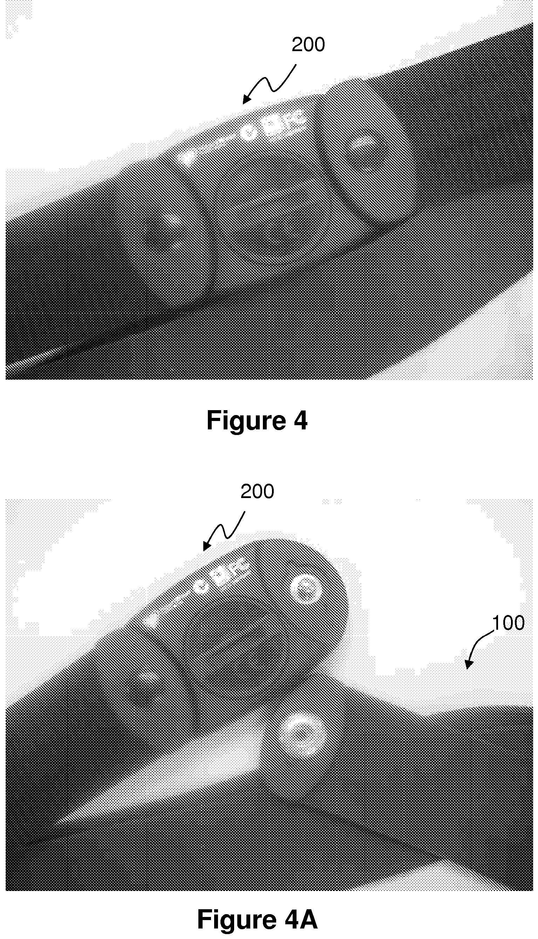

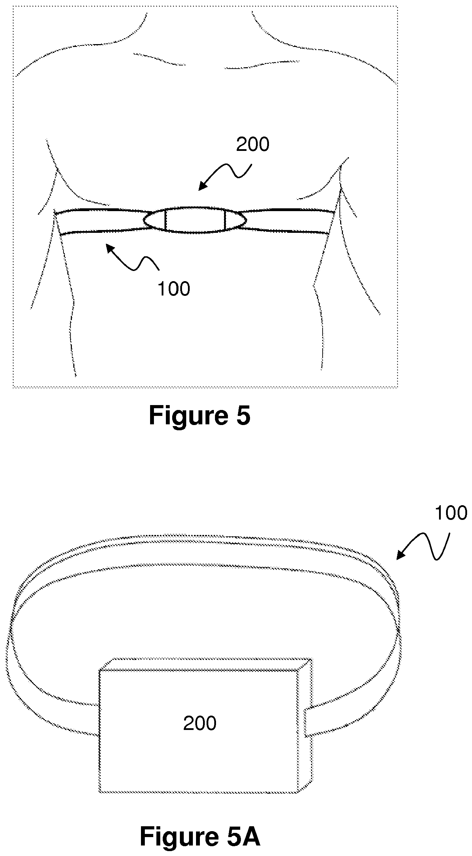

In an exemplary use of the chest strap as shown in Figures 4, 4A & 5, the chest strap is illustrated when used in combination with a mobile ECG signal processor 200. The ECG signal processor includes a rigid housing and a pair of signal input terminals. Each signal input terminal is a fastening component having a rigid mechanical mating portion which is configured to engage with a corresponding fastening component of the chest strap. The distance between the two signal collection surfaces in this application configuration therefore includes the longitudinal separation between the two fastening components on the mobile ECG signal processor. The ECG signal processor includes means for capturing and processing the detected ECG signals, as well as optional features for transmitting the relevant data to an external device for further processing or storage.

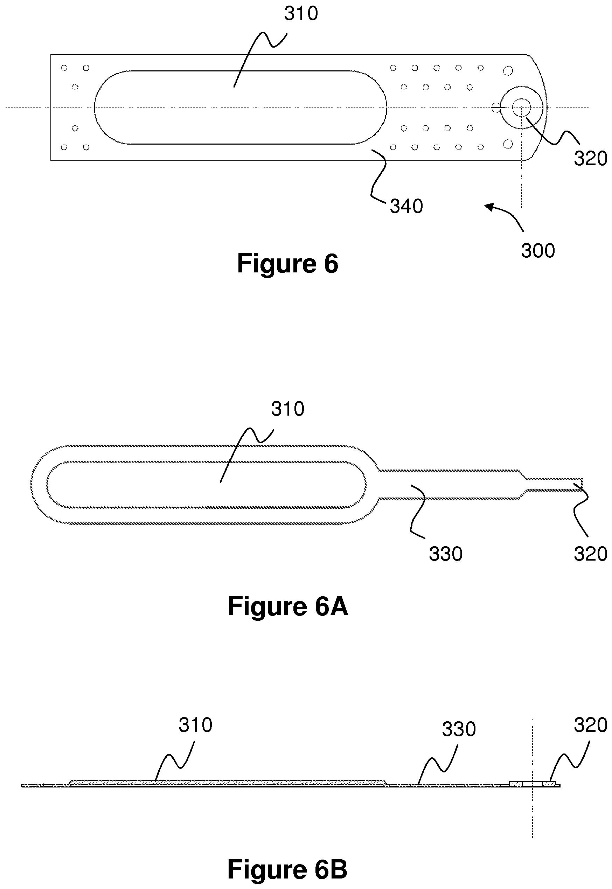

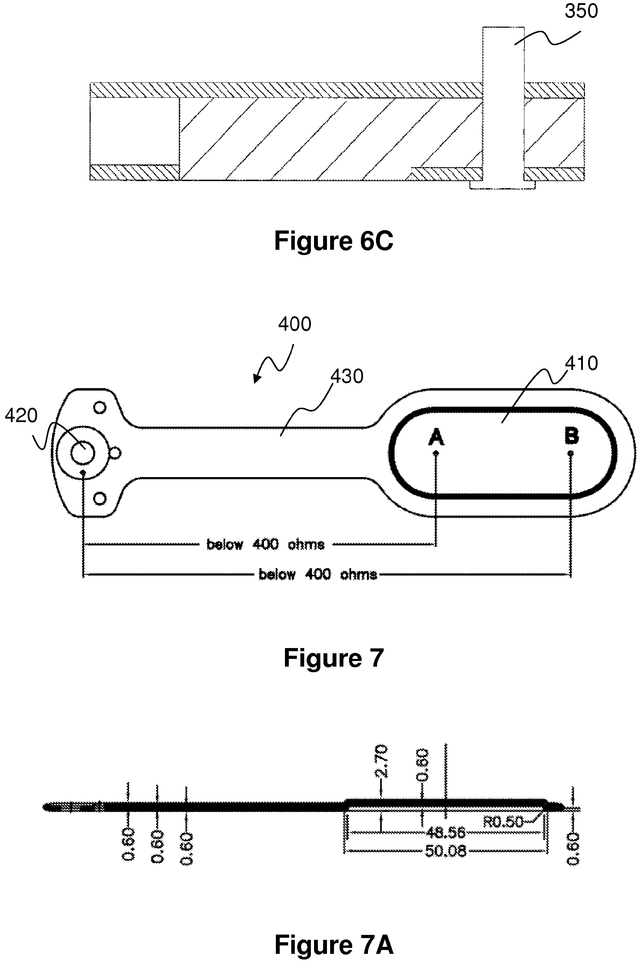

Referring to Figures 6 to 6C, there is shown an embodiment of a signal collection electrode 300 comprising a flexible contact pad 310, a signal output portion 320 and a bridging portion 330 interconnecting the flexible contact pad and the signal output portion. The signal collection electrode is integrally moulded of carbonized rubber and is substantially planar and elongate. As is better seen in Figures 6B and 6C, the flexible contact portion 310 protrudes above the bridging portion to form an elevated signal collection surface. The entire electrode, except for the signal collection surface and the signal output portion, is covered by an insulator, which is for example a moulded rubber sleeve 340. The rubber sleeve 340 comprises optionally perforations to improve permeability. A mechanical fastening component, for example a snap fastener part, is formed through the signal output portion to form the signal output terminal 350.

The electrode 400 of Figures 7 & 7A also comprises a flexible contact pad 410, a signal output portion 420 and a bridging portion 430 and is integrally moulded of a flexible and resilient conductive substance. The bridging portions of the electrodes of Figures 6 & 7 are narrower than the width of the signal collection surface. Such a narrowed bridging portion of a resilient material provides additional elasticity to strength the chest strap. In addition, the narrowed bridging portion also eliminates the need of extra breathing perforations.

As an alternative, the bridging portions of the electrodes could be of a same or comparable width to that of the signal collection surface, and perforations may be optionally formed outside the signal collection surfaces to improve breathability.

Regardless of the specific electrode designs, each of the signal collection pad is configured so that the effective area of the distributed signal collection surface could collect reasonable ECG signals for meaningful processing. The effective area here refers to the surface which exposes through the fabric strap and which has a typical width of about 1.5 to 2.5 cm and a typical length of about 5-8 cm. The resistance of the electrode, measured between the signal collection pad and the signal output portion, is typically below 1kΩ for good signal processing and operation.

In an exemplary use of the chest strap for swim applications, the electrode resistance is below 500Ω and preferably even below 400Ω. It is noted that such a low resistance provides a useful solution to enable ECG signal collection in both sea and chlorinated water. When using as a swim chest strap, the bridging portion is insulated by a moulded collar which is mould over the electrode, leaving only the signal collection surface and the signal output portion exposed. The insulated bridging portion mitigates signal leakage or interference when the chest is used in a highly conductive medium such as sea or chlorinated water.

While the present invention has been explained with reference to the embodiments above, it should be appreciated that the embodiments are only described to illustrate the invention and are not meant for restricting the scope of invention.

### Table of Numerals

| | | |
|---|---|---|
| 100 | | Chest strap |
| | 120 | First ECG signal collection electrode |
| | 140 | Second ECG signal collection electrode |
| | 180 | Strap |
| 200 | | Mobile ECG signal processor |
| 300 | | Signal collection electrode |
| | 310 | Contact pad |
| | 320 | Signal output portion |
| | 330 | Bridging portion |
| | 340 | Sleeve |
| | 350 | Signal output terminal |
| 400 | | Electrode |
| | 410 | Contact pad |
| | 420 | Output portion |
| | 430 | Bridging portion |

## Claims

1. A swim chest strap comprising a first ECG signal collection electrode and a second signal collection electrode for collection of ECG signals in conductive water such as sea water or chlorinated water during swimming or water sports,
wherein each signal collection electrode (120, 140, 300, 400) comprises a signal collection pad (310, 410) having a skin contact portion, a signal output portion (320, 420) comprising a signal output pad which includes a signal output terminal, and an elongate bridging portion (330, 430) interconnecting the signal collection pad (310, 410) and the signal output pad;
wherein the signal collection pad, the signal output pad and the bridging portion (330, 430) are integrally moulded of carbonized rubber which is a flexible, conductive and resilient material;
wherein the signal output portion is exposed for making external electrical contact and comprises a signal output terminal for outputting collected ECG signals; and wherein resistance between the skin contact portion and the signal output terminal is less than 500 ohms.

2. The strap according to Claim 1, wherein the signal output pad (320, 420) defines a signal output aperture, and the width of the bridging portion (330, 430) is smaller than that of the signal output pad (320, 420) but comparable to that of the signal output aperture; and wherein the signal output terminal (350) is a rigid conductive fastener formed through the signal output pad (320, 420).

3. The strap according to Claims 1 or 2, wherein the bridging portion (330, 430) is covered by a flexible, resilient and waterproof insulating material such as rubber, silicone rubber or soft plastics, the skin contact portion is elevated from the signal collection pad (310, 410) and surrounded by an insulating material, and the insulating material is over-moulded onto the electrode; wherein the signal output terminal comprises a mechanical mating portion of a conductive mechanical fastening part, and wherein the mechanical fastening parts of the first and second signal collection electrodes are adapted for mechanical and electrical connection with a portable ECG signal processing device.

4. The strap according to any of the preceding Claims, wherein effective resistance of the electrode measured between the skin contact portion and the signal output terminal is less than 400Ω.

5. The strap according to any of the preceding Claims, wherein the skin contact portion is flexible and elongate, has an effective skin contact surface area of between 7.5 cm² and 20cm², is between 1.5 to 2.5 cm wide and 5 to 8 cm long, and wherein the narrowed bridging portion (330, 430) is between 6 and 10 cm long and about half or less than half of the width of the skin contact portion.

6. The strap according to any preceding claim, wherein the signal output terminals (350) are riveted through the signal output pad to form the signal output portion.

7. The strap according to any preceding claim, wherein the first and second ECG signal collection electrodes (120, 140, 300, 400) are mounted on an insulating strap, the insulating strap having a first longitudinal end and a second longitudinal end, wherein the first and second ECG signal collection electrodes (120, 140, 300, 400) are mounted at or proximal to the first and second free longitudinal ends of the insulating strap (100); wherein first and second mechanical fastening parts adapted for making electrical and mechanical engagement with a portable ECG signal processing device are provided respectively at the first and second longitudinal ends of the strap for converting the longitudinal strap into a strap loop for wearing on the body of a user; and that ECG signals collected by the electrodes are delivered to the portable ECG signal processing device via the mechanical fastening parts.

8. The strap according to Claim 7, wherein the signal output terminal comprises a mechanical mating portion of a conductive mechanical fastening part which is adapted for engagement with a complementary mechanical mating portion of a counterpart mechanical fastening part and wherein each signal collection electrode comprises a signal output pad (320, 420) on which there is formed the signal output terminal and an elongate bridging portion (330, 430) interconnecting the signal collection pad and the signal output pad (320, 420); wherein the signal collection pad, the signal output pad (320, 420)and the bridging portion (330, 430) are integrally moulded of a flexible, conductive and resilient material; and wherein the signal output terminal is mechanically mounted on the signal output pad (320, 420) and formed as a mechanical mating portion of a conductive mechanical fastening part.

9. The strap according to Claims 7 or 8, wherein the first and second mechanical fastening parts of the strap are adapted for mechanical engagement with a portable ECG signal processing device, and to form a complete strap loop upon engagement with the portable ECG signal processing device, and wherein the strap is arranged such that ECG signals collected by the electrodes are delivered to the portable ECG signal processing device via the mechanical fastening parts.

10. The strap according to any of Claims 7 to 9, wherein the insulating strap is made of air and/or moisture permeable elastomeric fabric such as elastomeric polyamide or swimwear fabric such as Nylon, Lycra^{®}, polyester, or the like; and anti-slid texture is formed on portions of the insulating strap which are in contact with user skin during use.

11. The strap according to any of Claims 7 to 10, wherein the signal collection electrode is an electrode according to any of Claims 1 to 5, the bridging portion (330, 430) of each of the electrodes is embedded within the insulated strap, and the signal collection pad of each of the electrodes is bonded on the insulated strap.

12. The strap according to any of Claims 7 to 11, wherein the insulating strap is an elastic tensioning strap, and the signal output terminals of the first and second signal collection electrodes are the end terminals of the tensioning strap; and wherein the tensioning strap comprises mechanical fastening components located at longitudinal ends of the tensioning strap, and wherein the mechanical fastening components are arranged to convert the longitudinal tensioning strap into a tensioning strap loop; and wherein the conductive parts of the mechanical fastening components form part of the signal output terminals and/or vice versa.

13. A swim performance monitor comprising a swim motion detector arranged to detect swim motion data, an ECG signal processing device arranged to process physiological data of a user, and a strap according to any of Claims 7 to 12; wherein the swim motion detector and the ECG signal processing device are formed as a module and are housed within a common housing, and the ECG signal processing device has first and second signal input terminals; and wherein the ECG signal processing device is arranged to obtain ECG signals from the strap via the signal output terminals of the strap.

14. A swim performance monitor according to Claim 13, wherein resistance of the electrode is such that the effective signal path resistance between the skin contact portion of the electrode and a corresponding output terminal is less than that of the swim water signal path between the skin in contact with the electrode and the corresponding output terminal; and wherein the monitor is adapted for use in sea water, chlorinated water or other ionized water, and the effective signal path resistance of each of the electrodes is below 800 ohms, preferably below 500 ohms.

15. A swim performance monitor according to Claims 13 or 14, wherein each of the first and second terminals of the ECG processing device is formed as a mating portion of a mechanical fastener part, and each of the output terminals of the strap is formed into a complementary mating portion of a counterpart mechanical fastener part.

16. A physiological signal monitoring apparatus comprising a strap according to any of Claims 1 to 12 and a physiological signal processing module, the physiological signal processing module comprising a physiological signal processor arranged to process data collected by the strap; wherein the first electrode and the second electrode are arranged for collecting physiological signals from the human body for processing by the physiological signal processor; wherein the physiological signal processor is for processing ECG or heart rate signals and the first and second electrodes are arranged for collecting ECG or heart rate signals.

## Patentansprüche

1. Schwimmbrustgurt, umfassend eine erste EKG-Signalerfassungselektrode und eine zweite Signalerfassungselektrode zum Erfassen von EKG-Signalen in leitfähigem Wasser wie z.B. Meerwasser oder gechlortes Wasser während des Schwimmens oder Wassersports,
wobei jede Signalerfassungselektrode (120, 140, 300, 400) ein Signalerfassungspad (310, 410) mit einem Hautkontaktbereich, einen Signalausgabeteil (320, 420), der ein Signalausgabepad umfasst, das einen Signalausgangsanschluss beinhaltet, und einen länglichen Brückenteil (330, 430), der das Signalerfassungspad (310, 410) und das Signalausgabepad miteinander verbindet, umfasst;
wobei das Signalerfassungspad, das Signalausgabepad und der Brückenteil (330, 430) einteilig aus karbonisiertem Gummi geformt sind, der ein flexibles, leitfähiges und elastisches Material ist;
wobei der Signalausgabeteil für die Herstellung eines externen elektrischen Kontakts zugänglich ist und einen Signalausgangsanschluss zur Ausgabe erfasster EKG-Signale umfasst; und wobei der Widerstand zwischen dem Hautkontaktbereich und dem Signalausgangsanschluss weniger als 500 Ohm beträgt.

2. Schwimmbrustgurt gemäß Anspruch 1, wobei das Signalausgabepad (320, 420) eine Signalausgabeöffnung definiert und die Breite des Brückenteils (330, 430) kleiner als die des Signalausgabepads (320, 420), jedoch vergleichbar mit der der Signalausgabeöffnung ist; und wobei der Signalausgangsanschluss (350) ein durch das Signalausgabepad (320, 420) hindurch gebildetes starres leitfähiges Befestigungselement ist.

3. Gurt gemäß Anspruch 1 oder 2, wobei der Brückenteil (330, 430) von einem flexiblen, elastischen und wasserdichten Isoliermaterial wie z.B. Gummi, Silikonkautschuk oder Weichkunststoff bedeckt wird, der Hautkontaktbereich vom Signalerfassungspad (310, 410) abgehoben und von einem Isoliermaterial umgeben ist, und das Isoliermaterial auf die Elektrode aufgeformt ist; wobei der Signalausgangsanschluss ein mechanisches Verbindungsteil eines leitfähigen mechanischen Befestigungsteils umfasst, und wobei die mechanischen Befestigungsteile der ersten und der zweiten Signalerfassungselektrode für die mechanische und elektrische Verbindung mit einer tragbaren EKG-Signalverarbeitungsvorrichtung geeignet sind.

4. Gurt gemäß einem der vorhergehenden Ansprüche, wobei der effektive Widerstand der Elektrode, gemessen zwischen dem Hautkontaktbereich und dem Signalausgangsanschluss, weniger als 400 Ω beträgt.

5. Gurt gemäß einem der vorhergehenden Ansprüche, wobei der Hautkontaktbereich flexibel und länglich ist, eine effektive Hautkontaktoberfläche zwischen 7,5 cm² und 20 cm² besitzt, zwischen 1,5 und 2,5 cm breit und 5 bis 8 cm lang ist, und wobei der verengte Brückenteil (330, 430) zwischen 6 und 10 cm lang ist und etwa die halbe Breite oder weniger als die halbe Breite des Hautkontaktbereichs besitzt.

6. Gurt gemäß einem vorhergehenden Anspruch, wobei die Signalausgangsanschlüsse (350) durch das Signalausgabepad genietet sind und so der Signalausgabeteil gebildet wird.

7. Gurt gemäß einem vorhergehenden Anspruch, wobei die erste und die zweite EKG-Signalerfassungselektrode (120, 140, 300, 400) an einem Isoliergurt befestigt sind, wobei der Isoliergurt ein erstes longitudinales Ende und ein zweites longitudinales Ende besitzt, wobei die erste und die zweite EKG-Signalerfassungselektrode (120, 140, 300, 400) an oder nahe dem ersten und dem zweiten freien longitudinalen Ende des Isoliergurts (100) befestigt sind; wobei an dem ersten und dem zweiten longitudinalen Ende des Gurts erste und zweite mechanische Befestigungsteile angebracht sind, die sich zur Herstellung einer elektrischen und mechanischen Verbindung mit einer tragbaren EKG-Signalverarbeitungsvorrichtung eignen, um den longitudinalen Gurt in einen Gurtring zum Tragen am Körper eines Anwenders umzuwandeln; und dass von den Elektroden erfasste EKG-Signale über die mechanischen Befestigungsteile an die tragbare EKG-Signalverarbeitungsvorrichtung übertragen werden.

8. Gurt gemäß Anspruch 7, wobei der Signalausgangsanschluss einen mechanischen Verbindungsteil eines leitfähigen mechanischen Befestigungsteils umfasst, der mit einem komplementären mechanischen Verbindungsteil eines mechanischen Befestigungsteil-Gegenstücks verbunden werden kann, und wobei jede Signalerfassungselektrode ein Signalausgabepad (320, 420), auf dem der Signalausgangsanschluss ausgebildet ist, und einen länglichen Brückenteil (330, 430), der das Signalerfassungspad und das Signalausgabepad (320, 420) miteinander verbindet, umfasst, wobei das Signalerfassungspad, das Signalausgabepad (320, 420) und der Brückenteil (330, 430) einteilig aus einem flexiblen, leitfähigen und elastischen Material geformt sind; und wobei der Signalausgangsanschluss mechanisch am Signalausgabepad (320, 420) befestigt und als mechanischer Verbindungsteil eines leitfähigen mechanischen Befestigungsteils ausgebildet ist.

9. Gurt gemäß Anspruch 7 oder 8, wobei der erste und der zweite mechanische Befestigungsteil des Gurts geeignet sind, um sich mechanisch mit einer tragbaren EKG-Signalverarbeitungsvorrichtung zu verbinden, und um beim Verbinden mit der tragbaren EKG-Signalverarbeitungsvorrichtung einen kompletten Gurtring zu bilden, und wobei der Gurt so gestaltet ist, dass von den Elektroden erfasste EKG-Signale über die mechanischen Befestigungsteile an die tragbare EKG-Signalverarbeitungsvorrichtung übertragen werden.

10. Gurt gemäß einem der Ansprüche 7 bis 9, wobei der Isoliergurt aus luft- und/oder feuchtigkeitsdurchlässigem elastomerem Stoff wie z.B. elastomerem Polyamid oder Schwimmbekleidungsstoff wie z.B. Nylon, Lycra^{®}, Polyester oder dergleichen hergestellt ist; und wobei sich an den Bereichen des Isoliergurts, die während des Gebrauchs die Haut des Anwenders berühren, eine Anti-Rutsch-Textur befindet.

11. Gurt gemäß einem der Ansprüche 7 bis 10, wobei die Signalerfassungselektrode eine Elektrode gemäß einem der Ansprüche 1 bis 5 ist, der Brückenteil (330, 430) einer jeden Elektrode im Isoliergurt eingebettet ist, und das Signalerfassungspad einer jeden Elektrode auf dem Isoliergurt gebunden ist.

12. Gurt gemäß einem der Ansprüche 7 bis 11, wobei der Isoliergurt ein elastischer Spanngurt ist und die Signalausgangsanschlüsse der ersten und der zweiten Signalerfassungselektrode die Endstücke des Spanngurts sind; und wobei der Spanngurt mechanische Befestigungskomponenten umfasst, die sich an longitudinalen Enden des Spanngurts befinden, und wobei die mechanischen Befestigungskomponenten so angeordnet sind, dass sie den longitudinalen Spanngurt in einen Spanngurtring umwandeln; und wobei die leitfähigen Teile der mechanischen Befestigungskomponenten Teil der Signalausgangsanschlüsse und/oder umgekehrt bilden.

13. Schwimmleistungsmonitor, umfassend einen Schwimmbewegungsdetektor, der so gestaltet ist, dass er Schwimmbewegungsdaten erfasst, eine EKG-Signalverarbeitungsvorrichtung, die so gestaltet ist, dass sie physiologische Daten eines Anwenders erfasst, und einen Gurt gemäß einem der Ansprüche 7 bis 12; wobei der Schwimmbewegungsdetektor und die EKG-Signalverarbeitungsvorrichtung als ein Modul ausgebildet sind und in einem gemeinsamen Gehäuse untergebracht sind, und wobei die EKG-Signalverarbeitungsvorrichtung erste und zweite Signaleingangsanschlüsse besitzt; und wobei die EKG-Signalverarbeitungsvorrichtung so gestaltet ist, dass sie vom Gurt über die Signalausgabeanschlüsse des Gurts EKG-Signale erhält.

14. Schwimmleistungsmonitor gemäß Anspruch 13, wobei der Widerstand der Elektrode derart ist, dass der effektive Signalwegwiderstand zwischen dem Hautkontaktbereich der Elektrode und einem zugehörigen Ausgangsanschluss kleiner ist als der des Schwimmwassersignalwegs zwischen der Haut in Kontakt mit der Elektrode und dem zugehörigen Ausgangsanschluss; und wobei der Monitor für die Verwendung in Meerwasser, gechlortem Wasser oder anderem ionisiertem Wasser geeignet ist und der effektive Signalwegwiderstand einer jeden Elektrode unter 800 Ohm liegt, vorzugsweise unter 500 Ohm.

15. Schwimmleistungsmonitor gemäß Anspruch 13 oder 14, wobei jeder der ersten und zweiten Anschlüsse der EKG-Verarbeitungsvorrichtung als Verbindungsteil eines mechanischen Befestigungsteils ausgebildet ist, und jeder der Ausgangsanschlüsse des Gurts zu einem komplementären Verbindungsteil eines mechanischen Befestigungsteil-Gegenstücks ausgebildet ist.

16. Physiologische Signalüberwachungsvorrichtung, umfassend einen Gurt gemäß einem der Ansprüche 1 bis 12 und ein physiologisches Signalverarbeitungsmodul, wobei das physiologische Signalverarbeitungsmodul einen physiologischen Signalprozessor umfasst, der so gestaltet ist, dass er vom Gurt gesammelte Daten verarbeiten kann; wobei die erste Elektrode und die zweite Elektrode so gestaltet sind, dass sie physiologische Signale vom menschlichen Körper für die Verarbeitung durch den physiologischen Signalprozessor erfassen können; wobei der physiologische Signalprozessor zur Verarbeitung von EKG- oder Herzfrequenzsignalen dient, und die erste und die zweite Elektrode so gestaltet sind, dass sie EKG- oder Herzfrequenzsignale erfassen können.

## Revendications

1. Sangle thoracique de natation comprenant une première électrode de collecte de signaux ECG et une seconde électrode de collecte de signaux pour la collecte de signaux ECG dans l'eau conductrice telle que l'eau de mer ou l'eau chlorée pendant la natation ou les sports nautiques,
dans laquelle chaque électrode de collecte de signaux (120, 140, 300, 400) comprend une pastille de collecte de signaux (310, 410) dotée d'une partie de contact avec la peau, d'une partie de sortie de signaux (320, 420) comprenant une pastille de sortie de signaux qui inclut une borne de sortie de signaux, et une partie de liaison allongée (330, 430) interconnectant la pastille de collecte de signaux (310, 410) et la pastille de sortie de signaux ;
dans laquelle la pastille de collecte des signaux, la pastille de sortie des signaux et la partie de liaison (330, 430) sont intégralement moulées en caoutchouc carbonisé, qui est un matériau souple, conducteur et résilient ;
dans laquelle la partie de sortie du signal est exposée pour établir un contact électrique externe et comprend une borne de sortie du signal pour émettre les signaux ECG recueillis ; et dans laquelle la résistance entre la partie de contact avec la peau et la borne de sortie du signal est inférieure à 500 ohms.

2. Sangle selon la revendication 1, dans laquelle la pastille de sortie de signal (320, 420) définit une ouverture de sortie de signal, et la largeur de la partie de liaison (330, 430) est plus petite que celle de la pastille de sortie de signal (320, 420) mais comparable à celle de l'ouverture de sortie de signal ; et dans laquelle la borne de sortie de signal (350) est une attache conductrice rigide formée à travers la pastille de sortie de signal (320, 420).

3. Sangle selon les revendications 1 ou 2, dans laquelle la partie de liaison (330, 430) est recouverte d'un matériau isolant flexible, résilient et imperméable tel que le caoutchouc, le caoutchouc de silicone ou les plastiques souples, la partie de contact avec la peau est surélevée par rapport à la pastille de collecte des signaux (310, 410) et entourée d'un matériau isolant, et le matériau isolant est surmoulé sur l'électrode ; dans laquelle la borne de sortie du signal comprend une partie d'accouplement mécanique d'une pièce de fixation mécanique conductrice, et dans laquelle les pièces de fixation mécanique des première et seconde électrodes de collecte des signaux sont adaptées à la connexion mécanique et électrique avec un dispositif portable de traitement des signaux ECG.

4. Sangle selon l'une quelconque des revendications précédentes, dans laquelle la résistance effective de l'électrode mesurée entre la partie en contact avec la peau et la borne de sortie du signal est inférieure à 400Ω.

5. Sangle selon l'une quelconque des revendications précédentes, dans laquelle la partie en contact avec la peau est flexible et allongée, présente une surface effective de contact avec la peau comprise entre 7,5 cm² et 20 cm², présente une largeur comprise entre 1,5 et 2,5 cm et une longueur comprise entre 5 et 8 cm, et dans laquelle la partie de liaison rétrécie (330, 430) présente une longueur comprise entre 6 et 10 cm et représente environ la moitié ou moins de la moitié de la largeur de la partie en contact avec la peau.

6. Sangle selon l'une quelconque des revendications précédentes, dans laquelle les bornes de sortie de signal (350) sont rivetées à travers la pastille de sortie de signal pour former la partie de sortie de signal.

7. Sangle selon l'une quelconque des revendications précédentes, dans laquelle les première et seconde électrodes de collecte de signaux ECG (120, 140, 300, 400) sont montées sur une sangle isolante, la sangle isolante présentant une première extrémité longitudinale et une seconde extrémité longitudinale, dans laquelle les première et seconde électrodes de collecte de signaux ECG (120, 140, 300, 400) sont montées au niveau ou à proximité des première et seconde extrémités longitudinales libres de la sangle isolante (100) ; dans laquelle une première et une seconde pièces de fixation mécanique adaptées pour s'engager électriquement et mécaniquement avec un dispositif portable de traitement des signaux ECG sont fournies respectivement à la première et à la seconde extrémité longitudinale de la sangle pour convertir la sangle longitudinale en une boucle de sangle à porter sur le corps d'un utilisateur ; et dans laquelle les signaux ECG recueillis par les électrodes sont délivrés au dispositif portable de traitement des signaux ECG par l'intermédiaire des pièces de fixation mécanique.

8. Sangle selon la revendication 7, dans laquelle la borne de sortie de signal comprend une partie d'accouplement mécanique d'une pièce de fixation mécanique conductrice qui est adaptée pour Se mettre en prise avec une partie d'accouplement mécanique complémentaire d'une pièce de fixation mécanique homologue et dans laquelle chaque électrode de collecte de signal comprend une pastille de sortie de signal (320, 420) sur laquelle est formé le terminal de sortie de signal et une partie de liaison allongée (330, 430) interconnectant la pastille de collecte de signal et la pastille de sortie de signal (320, 420) ; dans laquelle la plaque de collecte des signaux, la plaque de sortie des signaux (320, 420) et la partie de liaison (330, 430) sont intégralement moulées dans un matériau flexible, conducteur et résilient ; et dans laquelle la borne de sortie des signaux est montée mécaniquement sur la plaque de sortie des signaux (320, 420) et formée en tant que partie d'accouplement mécanique d'une pièce de fixation mécanique conductrice.

9. Sangle selon les revendications 7 ou 8, dans laquelle les première et seconde parties de fixation mécanique de la sangle sont adaptées à la mise en prise mécanique avec un dispositif portable de traitement des signaux ECG, et pour former une boucle de sangle complète lors de la mise en prise avec le dispositif portable de traitement des signaux ECG, et dans laquelle la sangle est disposée de telle sorte que les signaux ECG recueillis par les électrodes sont délivrés au dispositif portable de traitement des signaux ECG par l'intermédiaire des parties de fixation mécanique.

10. Sangle selon l'une quelconque des revendications 7 à 9, dans laquelle la sangle isolante est faite d'un tissu élastomère perméable à l'air et/ou à l'humidité, tel qu'un polyamide élastomère ou un tissu pour maillots de bain tel que du nylon, du Lycra^{®}, du polyester ou autre ; et une texture antidérapante est formée sur les parties de la sangle isolante qui sont en contact avec la peau de l'utilisateur lors de l'utilisation.

11. Sangle selon l'une quelconque des revendications 7 à 10, dans laquelle l'électrode de collecte de signaux est une électrode selon l'une quelconque des revendications 1 à 5, la partie de liaison (330, 430) de chacune des électrodes est insérée dans le bracelet isolé, et la pastille de collecte de signaux de chacune des électrodes est collée sur le bracelet isolé.

12. Sangle selon l'une quelconque des revendications 7 à 11, dans laquelle la sangle isolante est une sangle de tension élastique, et les bornes de sortie de signal des première et seconde électrodes de collecte de signal sont les bornes d'extrémité de la sangle de tension ; et dans laquelle la sangle de tension comprend des composants de fixation mécanique situés aux extrémités longitudinales de la sangle de tension, et dans laquelle les composants de fixation mécanique sont disposés pour convertir la sangle de tension longitudinale en une boucle de sangle de tension ; et dans laquelle les parties conductrices des composants de fixation mécanique font partie des bornes de sortie de signal et/ou inversement.

13. Système de surveillance de performances de natation comprenant un détecteur de mouvement de natation conçu pour détecter les données de mouvement de natation, un dispositif de traitement du signal ECG conçu pour traiter les données physiologiques d'un utilisateur, et un bracelet selon l'une quelconque des revendications 7 à 12 ; dans lequel le détecteur de mouvement de natation et le dispositif de traitement du signal ECG sont formés comme un module et sont logés dans un boîtier commun, et le dispositif de traitement du signal ECG présente une première et une seconde borne d'entrée de signal ; et dans lequel le dispositif de traitement du signal ECG est conçu pour obtenir des signaux ECG de la sangle par l'intermédiaire des bornes de sortie de signal de la sangle.

14. Système de surveillance de performances de natation selon la revendication 13, dans lequel la résistance de l'électrode est telle que la résistance effective du trajet du signal entre la partie de l'électrode en contact avec la peau et une borne de sortie correspondante est inférieure à celle du trajet du signal de l'eau de natation entre la peau en contact avec l'électrode et la borne de sortie correspondante ; et dans lequel le système de surveillance est adapté à une utilisation dans l'eau de mer, l'eau chlorée ou d'autres eaux ionisées, et la résistance effective du trajet du signal de chacune des électrodes est inférieure à 800 ohms, et de préférence inférieure à 500 ohms.

15. Système de surveillance de performances de natation selon les revendications 13 ou 14, dans lequel chacun des premiers et seconds terminaux du dispositif de traitement ECG est formé comme une partie d'accouplement d'une pièce de fixation mécanique, et chacun des terminaux de sortie de la sangle est formé comme une partie d'accouplement complémentaire d'une pièce de fixation mécanique homologue.

16. Appareil de surveillance des signaux physiologiques comprenant une sangle selon l'une quelconque des revendications 1 à 12 et un module de traitement des signaux physiologiques, le module de traitement des signaux physiologiques comprenant un processeur de signaux physiologiques conçu pour traiter les données recueillies par la sangle ; la première électrode et la seconde électrode étant conçues pour recueillir des signaux physiologiques du corps humain en vue de leur traitement par le processeur de signaux physiologiques ; le processeur de signaux physiologiques étant conçu pour traiter des signaux d'ECG ou de fréquence cardiaque et la première et la seconde électrodes étant conçues pour recueillir des signaux d'ECG ou de fréquence cardiaque.
